# EUROPEAN PATENT APPLICATION

(11) **EP 4 616 830 A2**
(43) Date of publication of application: **17.09.2025**
(21) Application number: 25160218.1
(22) Date of filing: 26.02.2025
(51) Int. Cl.: A61F 2/50, A61F 2/78, A61F 2/80

(54) **METHOD FOR THE PRODUCTION OF TRANSFEMORAL PROSTHESES, SYSTEM FOR IMPLEMENTING THE SAID METHOD AND TRANSFEMORAL PROSTHESIS**

(30) Priority: 26.02.2024 IT 202400003994
(71) Applicant: RO.GA. S.p.A., 94100 Enna (EN) (IT)
(72) Inventor: Colaleo, Stefano Rosario Maria, 94100 Enna (IT)
(74) Representative: Karaghiosoff, Giorgio Alessandro

(57) **Abstract**

Method of designing a trans-femoral prosthesis, which includes an artificial limb replacing the missing limb and a terminal for coupling said artificial limb to a stump, the method comprising the following steps:
the separation of the coupling terminal to the stump into two functional components (1, 2);
the definition of an algorithm that describes the three-dimensional trend of the shape of the internal surface of the first component (1) intended to adhere to the external surface of a generic stump;
generating a function that describes the shape of the internal surface of said first component (1) morphologically adapted to a specific stump;
the generation of a digital model of said first and/or second component configurable or convertible into a command file for the control of a manufacturing device of the numerical control type;
manufacturing of said prosthetic socket obtained by executing said command file; and
the coupling of the said two components of the said prosthetic socket.

## Description

### Technical field of the invention

The invention refers to the technical field of the production of prostheses and in particular of the prostheses of the lower and/or upper limbs such as in particular the prostheses called transfemoral, transtibial, or similar.

In transfemoral prostheses, approximately 80% of the body weight is transmitted to the socket by the ischial tuberosity. The remaining and minor part of the load is instead transferred to the walls of the socket via the stump muscles that are in contact with them. The pressure values on the socket become important due to the limited support surface. Consequently, to reduce these stresses, it is necessary to distribute them over the total surface of the stump and its terminal part.

If the socket is not well designed, a moment of lateral flexion can be generated on the femoral head and on the ischial support, with consequent asymmetry and instability in the patient's movement.

At the state of the art, there are two solutions for designing a socket for transfemoral prostheses:
- Quadrilateral: This solution allows you to obtain direct support of the ischial load. The socket of the prosthesis is in total contact with the stump to promote blood circulation in the stump and venous return, avoiding the formation of edema. Furthermore, the increased support area favors the distribution of pressure points, so as to guarantee greater comfort to the subject during movement. This type of socket is elongated in the medio-lateral direction and narrow in the antero-posterior direction and often does not respect the physiology of the thigh muscles, which can lead to adverse events such as a tendency towards hypotrophy (reduction in the size of a tissue due to a decrease in volume), pain in the ischial-perineal area and at the femoral apex;
- With ischial containment: This solution has an inclined postero-medial edge to support the ischial tuberosity. The orientation and containment shape of the ischial support determines a lower impact on the ischial tuberosity and a lower reduction in blood flow. The medial grip on the ischial ramus reduces the relative movement between the stump and the socket. As a result, greater stability is achieved during movement, limiting any lateral slippage of the socket and any pressure on the femoral apex. The lateral sub-trochanteric and medial thrusts on the ischial branch, together with the compression of the soft parts, are directed towards the center of the hip and result in a vertical force that passes through the femoral head; this force adds to the foot-ground reaction, limiting the moments on the frontal plane which cause instability.

As regards the manufacturing of said prostheses, in order to create sockets to house the stumps, various state-of-the-art manufacturing processes are used.

A first known manufacturing process involves making a cast of the stump using bandages and plaster and building a mold on said model for manufacturing a corresponding socket.

This method has several disadvantages as it is a purely artisanal process and strongly depends on the experience and skills of the person carrying out the mold formation process. Therefore, the process always requires the presence of the personnel in charge of the patient and therefore presents high costs. Additionally, in case of an error, the process must be aborted and a new cast manufacturing process must be started.

In addition to the formation of the cast, the manufacturing of the socket corresponding to the said cast is also an essentially manual process and is subject to variations in customization on the respective patient.

The entire process is relatively long and often takes up to several days before the reservoir can be ready for use.

At the state of the art, it is also known to use 3D scanners which acquire the shape of the stump in the form of a three-dimensional cloud of points and from which it is possible to extrapolate the control commands of material processing machines for the creation of the socket of the prosthesis.

The 3D scanner also has several drawbacks. Firstly, the device is associated with a certain relatively high cost, so the tool is not always available. From a purely functional point of view, the 3D scanner captures only the external points, i.e. the conformation of the surface and does not allow the collection of information relating to the various components such as the bone components and/or the landmarks which are relevant for the conformation of the socket. Furthermore, the collected data requires time-consuming processing and post processing of the collected point cloud and its model in order to use the data to control manufacturing machines.

One of the main purposes of a socket is to ensure an excellent fit. This requires the development of a system capable of managing the changes in volume and shape to which the residual limb is subjected throughout the day. If the volume of the stump increases, the consequence is an increase in pressures and shear stresses at the socket-stump interface which lead to problems in the surrounding tissues. For this reason, the socket represents one of the most studied elements, as the development of a socket capable of adapting to variations in volume would guarantee continuous comfort for the user.

Abnormal stress conditions and temperature variations at the socket-stump interface are among the factors triggering variations in the volume of the residual limb (shrinkage or expansion). A 3.5% variation in volume is sufficient to cause the patient to feel unwell. It is estimated that during the day the stump can undergo a reduction of approximately 10% of its volume. Therefore, optimizing the fit of the socket by taking into account the volumetric variations of the stump represent a fundamental objective for improving the quality of life of amputees.

### Summary of the invention

The present invention aims to create a method for the design and subsequent production of a socket for amputees of the lower and/or upper limbs, in particular for transfemoral, trans-tibial and/or similar amputees, which guarantees the overcoming of state-of-the-art solutions and the management, directly by its user, of variations in the volume of the stump, always maintaining optimal configurations of the mutual support surfaces of the interface between the stump and the prosthetic socket.

According to a further aspect, the present invention aims at a method for the anatomically dedicated design of the socket with respect to the anatomical configuration of the stump, without requiring techniques for acquiring the morphological conformation of the said stump which require long execution times and also a considerable number of personnel specifically trained in the execution of part of the steps of the said design method.

According to yet another aspect of the present invention, it aims to create a method of designing the socket in a way that morphologically corresponds to the morphological configuration of a stump, which method can be envisaged in combination with a manufacturing process of the said socket.

The present invention also aims to create a system for implementing said method.

Furthermore, the present invention aims at a transfemoral prosthesis which has a construction such as to overcome the drawbacks of prostheses known in the state of the art and, in particular, which is morphologically optimized to the stump with regard to the interface surface of the prosthetic socket with said stump and which is also adjustable over time, compared to an optimal initial setting, directly by the patient in order to adapt the shape of the socket to the variations of the residual limb, i.e. of the stump, to obtain an optimal condition of resting the surface of the stump on the internal surface of the socket itself.

According to yet another aspect, the present invention aims to make adjustable the position of the socket in relation to the terminal resting on the ground, to an extent compliant with the needs of correct posture.

The present invention achieves the aforementioned objectives with a method of designing a trans-femoral prosthesis, which trans-femoral prosthesis includes an artificial limb replacing the missing limb and a terminal for coupling said artificial limb to a stump, which terminal is in the form of an anatomically shaped socket on the stump and in which the said stump is housed and which can be fixed to the upper end of the said artificial limb, which method involves the following steps:
the separation of the coupling terminal to the stump into two functional components which can be coupled and/or separated from each other and of which,
a first component of flexible material and a second component which is constituted by a support structure of rigid or less flexible material than that of the first component and which second component has a housing cavity for said first component of flexible material;
providing said first component with a shape defining a cavity delimited by a wall which has an internal surface and an external surface spaced one from the other by a thickness of the said wall;
the housing cavity of the said second component for the said first component having an internal surface corresponding to the shape of at least some areas of the external surface of the said first component, at least some of the said areas of the external surface of the said first component being provided for being overlapped by the said internal surface of the said second component;
defining an algorithm that describes the three-dimensional trend of the shape of the internal surface of the first component intended to adhere to the external surface of a generic stump on the basis of descriptive parameters of pre-established dimensional sizes of said stump;
generating a function that describes the shape of the internal surface of said first component morphologically adapted to a specific stump, applying said algorithm to values of said descriptive parameters of said pre-established dimensional quantities measured on said specific stump;
generating a digital model of the internal surface and of the external surface of at least the said first component of flexible material and/or of the internal surface of said second component of more rigid material at the said at least some areas basing on the said function, which digital model is configurable or convertible into a command file for the control of a numerically controlled manufacturing device, such as a 3D printer or chip removal machine, or the like,
the manufacturing of said prosthetic socket obtained by executing said command file;
the coupling of the said two components of the said prosthetic socket, i.e. the support structure of rigid or less flexible material and the first component of flexible material;
the fixing to said supporting structure to an artificial substitute for the missing limb.

It has to be noticed that once the internal shape of the cavity for the stump in the first component has been defined, then basing on this data it is possible to generate also the external shape of the said first component and the corresponding internal shape of the second component, at least in the areas of contact of the first component with the said second component. Indeed, providing thickness data of the wall of the first component, these data define the shape and the position of the shape of the external surface of the first component relatively to the internal shape of the said first component. Once the external shape of the first component has been defined, it is a straightforward step to determine the internal shape of the second component at least at the areas of contact of the said second component with the said first component when said first component is fitted inside the housing cavity of the said second component.

Although the steps of the aforementioned method are reported and described in relation to a transfemoral prosthesis, the expert in the field is able to adapt the essential steps of the method to apply them to the creation of other types of prostheses relating to the lower and/or upper limbs.

According to a preferred embodiment of the method, the said method provides for the creation of the said rigid or less flexible structure in the form of a deformable element relative to a dimensional variation in at least one preferably in a plurality of pre-established directions relative to a longitudinal axis of the said rigid structure.

According to a specific embodiment, the method involves the use of an algorithm that describes the shape of the external surface of the stump on the basis of dimensional parameters of the said stump.

According to a specific embodiment, the algorithm is configured to calculate the shape of the external surface of a specific stump and therefore of the corresponding internal surface of the socket on the basis of the dimensional values of a stump relating to one or more of the following parameters: right or left limb, length of the stump, measurement of the thigh, circumference of the stump, thickness of the stump in pre-established points of its axial extension, any inclinations of the circumferences with respect to the horizontal orientation and furthermore of the height and weight of the user and or further based on one or more parameters relating to the device for anchoring the socket to the artificial limb.

Still according to a characteristic of the method according to one or more of the embodiments described above, the support or load bearing structure includes elements for variable radial compression of the flexible socket against the stump, which elements are provided in at least one circumferential band of the said stump and/or elements for localized compression of at least a partial area of the socket against the stump and which elements are connected to each other by means of relative displacement members which can be controlled manually in the direction of mutual approach and/or in the direction of separation, the said algorithm being configured to calculate the position of the mutual displacement members of the localized compression elements that form the said support or load bearing structure and an initial compression condition, while a manual control of the compression condition is provided by activating the said reciprocal displacement members in order to manage the volumetric change during the day, the initial compression condition calculated by the algorithm being defined to an extent such as to regulate the vascular and lymphatic pressure of the stump, to prevent it from swelling and the compression condition being regulated manually in order to improve the comfort of the fit.

In one embodiment, the said relative movement members are constituted by tie rods and/or strings which are sent back to at least one mechanism for winding and/or unwinding the said tie rods on a reel.

In one embodiment, a single tie rod is provided which is guided along the structure and connects all the elements that make up said structure to each other in succession.

In an exemplary embodiment, the tie rod(s) and the winding and unwinding member or the winding and unwinding members are made up of devices, for example, and not limited to, of the type known as BOA System from the company Boa LLC.

In one embodiment, an organ is provided for regulating the relative coupling position of the said socket with the said artificial limb, which organ allows the relative position of the said socket and the said artificial limb to be varied according to at least two degrees of freedom, according to at least two perpendicular directions in the horizontal plane.

From the above description, the advantages of the method according to the present invention appear evident. The parameterized algorithm that describes the three-dimensional shape of the stump in relation to the interface surface with the internal surface of the prosthetic socket allows the generation of a standard shape of the said surfaces which is adapted to a specific stump by applying the said algorithm to the values of the parameters measured on the specific stump. The algorithm thus calculates a function that represents the said three-dimensional shape and which in turn can be easily converted into a print file for 3D printers.

The parameters required for determining the specific three-dimensional shape of the surface of a stump and therefore of the corresponding internal 3D surface of the socket are made up of simple dimensional parameters which require technical personnel without particular experience for the measurement.

The calculation of the specific shape of the said 3D surface is quick and easy, as is the production of the socket using 3D printing techniques.

Furthermore, the provision of a breakdown into two different functional components of the prosthesis, i.e. the prosthetic socket of flexible material and the support or load bearing structure of more rigid material, allows the algorithm to calculate the configuration of the combination of variable compression elements on the stump of said socket and of the mutual displacement members of said variable compression elements.

An advantageous construction of the rigid structure is made up of a basic set of compression elements of a standardized type which are intended to be modified by the algorithm in relation to some shape parameters and which are intended to be articulated together or connected in such a way as to be movable, said elements being optionally modified in relation to the dimensions and which elements are mutually movable thanks to the relative displacement members, thus making said external structure adjustable in relation to the dimensions of the housing compartment of the socket This allows to have the same combination of variable compression elements for the support structure for the different sockets and which, thanks to the movement members, are positioned together in a morphologically corresponding way to the specific stump thanks to the configuration of said prosthetic socket according to the steps described above.

The present invention is also directed to a system for the design and production of a transfemoral prosthesis and in particular of a terminal for coupling the same to a stump according to the present invention.

According to a more general embodiment, the said system includes:
at least one processing unit, which processing unit comprises at least one memory in which a program is stored or can be stored which encodes the instructions for the execution of a parameterized algorithm for calculating the function of representing a three-dimensional shape of the external surface of a stump and of the corresponding internal surface of a first component of the socket in which said stump is intended to be housed;
at least one interface for entering measurement data of the values of said parameters which have been detected by measuring a specific stump;
at least one output interface of a function that describes the three-dimensional shape of the said specific stump and of the corresponding internal surface of the said component of flexible material of the socket, which function is calculated by executing the algorithm with the measured values of the said parameters;
and optionally which algorithm also describes the three-dimensional shape of a support structure which overlaps from the outside to the said first component of flexible material of the socket and is made up of a plurality of variable compression elements adjustable between them in relation to their mutual position and also which describes the position and/or type of relative displacement members of the said variable compression elements between each other;
at least one converter which converts the said descriptive functions of the said three-dimensional surface and/or of the said support structure into a command file of a manufacturing device such as a 3D printer and/or of molding devices and/or of chip removal processing devices, for the manufacturing of a socket with a first component of flexible material which presents a recess with an internal surface which has a shape corresponding to that described by the aforementioned function and/or for the manufacturing or configuration of the said support structure support, or the elements that constitute it, or the relative displacement members of the variable compression members that constitute the said support structure;
at least one device for coupling the said first component of flexible material of the said socket with the said second component which is constituted by the said support structure, which support structure includes a plurality of separate elements which externally cover the said prosthetic socket and can be approached and/or moved away from each other.

The invention is directed also to a prosthesis, for example, a transfemoral one, which prosthesis comprises an artificial limb to replace the missing limb and a terminal for coupling said artificial limb to a stump, which terminal can be fixed to the upper end of said artificial limb and which terminal for coupling to the stump is constituted by a combination of at least two functionally cooperating components,

Which prosthesis is manufactured with a method according to one or more of the preceding embodiments or variants and optionally with a system as described above and
wherein the prosthetic socket is formed by the combination of:
a first component of flexible material and a second component which is constituted by a support structure of rigid or less flexible material than that of the first component and which second component has a housing cavity for said first component of flexible material;
said first component being provided with a cavity delimited by a wall which has an internal surface and an external surface spaced one from the other by a thickness of the said wall;
said housing cavity of the said second component for the said first component having an internal surface corresponding to the shape of at least some areas of the external surface of the said first component, at least some of the said areas of the external surface of the said first component being provided for being overlapped by the said internal surface of the said second component;
said second component is provided with at least one upper opening of the said housing cavity for insertion of the said first component and with a closed bottom element having a concave internal surface;
said second component further comprising a lateral leg element and an opposing medial leg element which are hinged at their lower edge to the upper edge of said bottom element;
said two leg elements having an angular extension in the circumferential direction smaller than the entire circumferential extension of the external surface of the first component;
said two leg elements being connected to each other at a substantially circumferential band provided in the area of the upper half of the support structure by means of tie rods combined with traction organs for variable tightening of the said two leg elements one against the other and against the first component and by means of circumferential guiding plates engaging both leg elements in a circumferentially sliding way in sliding guides provided on the said leg elements at the mutual facing edges of the said leg elements, said guides being preferably provided on the internal side of the said leg elements facing the external surface of the first component and
the tie rods are passing on the said circumferential plates with circumferential sections thereof.

According to a further feature, the second component, i.e. at least the said leg component, are composed of several elements separated from each other and joined by one or more mutual clamping elements, of which one or more mutual clamping elements are connected to one or more manually controlled shortening and/or lengthening members and in which optionally and said one or more members can be constituted for example by one or more winding cores of at least one of the said clamping elements and the said clamping elements are in the form of one or more flexible tie rods.

In an embodiment, said first component of flexible material and the said second more rigid component constituted by the support structure are provided with mutual engagement elements for centering said first component with respect to the said second component which are preferably provided in the area of the bottom element of the support or load bearing structure and of the corresponding closed lower end of the first component (1).

According to an embodiment, the flexible component has an internal surface in contact with the external surface of a specific stump which is configured in an anatomically corresponding way with the contact surface of the said stump by calculating a function which describes the said surfaces, i.e. the external one of the stump and the corresponding internal one of the housing provided in the first component by means of a parametric algorithm for configuring the said function, which parameters are constituted by one or more dimensional parameters of the said stump and being at least the said first component and/or also the said second component of the said socket made using a numerically controlled manufacturing device, such as a 3D printer and/or a processing machine for chip removal and/or a deformation using an at least partially configurable forming mold, which manufacturing devices are controlled using as the command file of the said printer a file deriving from the conversion of the said representation function of the said internal surface of the said prosthetic socket.

According to yet a further feature, the first and second components are provided with a corresponding shape, whereby the said second component externally covers the said first component for a substantial part of the external surface of the said first component.

According to one feature, the said support or load-bearing structure is composed of at least two parts, preferably of several elements separated from each other and joined by one or more mutual clamping elements, which one or more mutual clamping elements are connected to one or more manually controlled shortening and/or lengthening members of the said clamping elements.

The said one or more members can be constituted for example by one or more winding cores of at least one of the said tightening elements and the said tightening elements are in the form of one or more flexible tie rods, such as for example and not limited to one or more laces associated with one or more winding and unwinding devices, for example, of the type called Boa System.

In the present description and in the claims the term Support structure, supporting structure and load bearing structure all refers to the sabe part and has to be considered having an identical meaning in relation to the invention.

Further features and advantages are the subject of the dependent claims.

### Brief description of the figures

An executive, non-limiting example of the invention is described below with reference to the attached figures, in which:

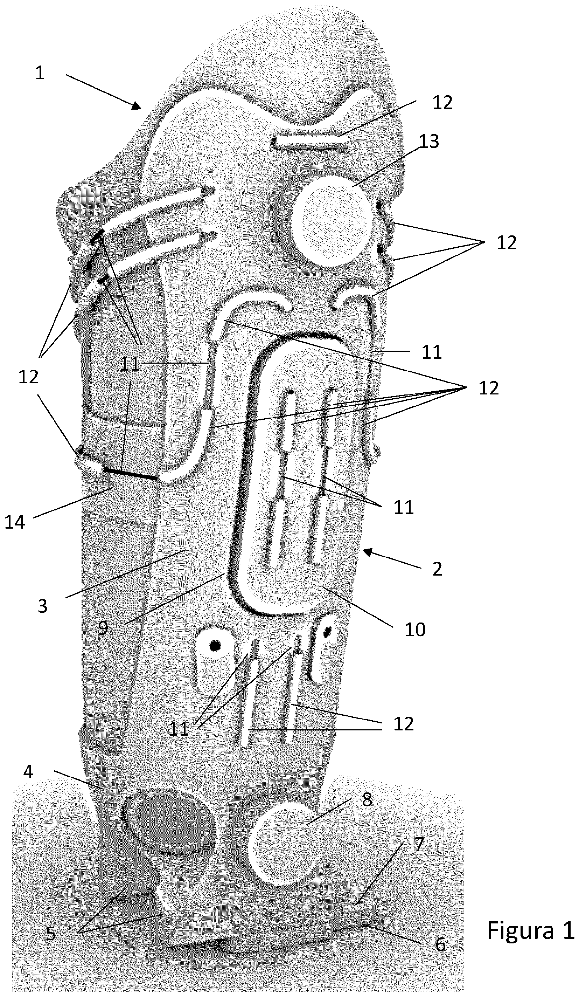

Figure 1 shows a perspective view on the anterior-lateral side of a coupling terminal of a complete transfemoral prosthesis according to an example of the present invention.

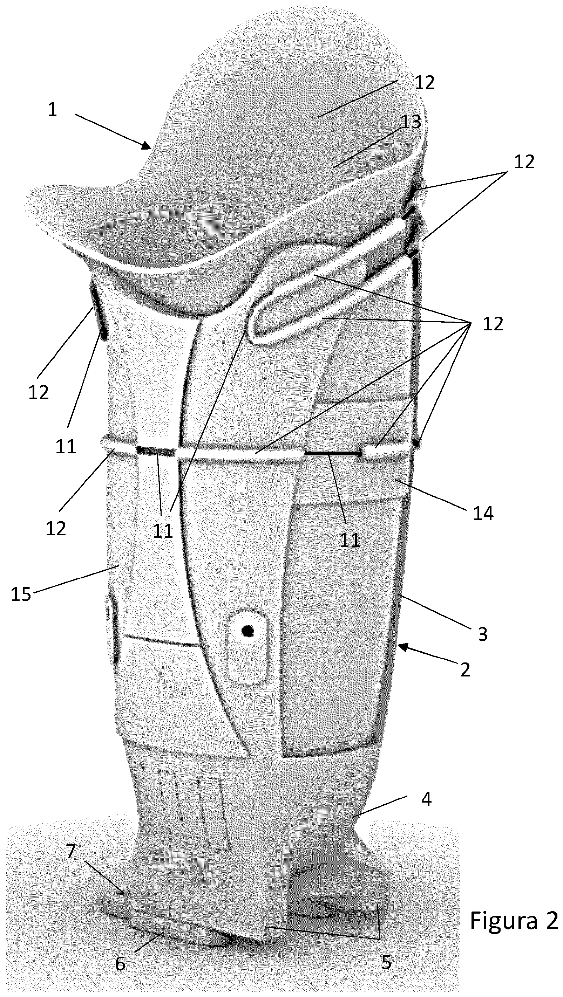

Figure 2 shows a perspective view on the anterior-medial side of the stump coupling terminal according to Figure 1.

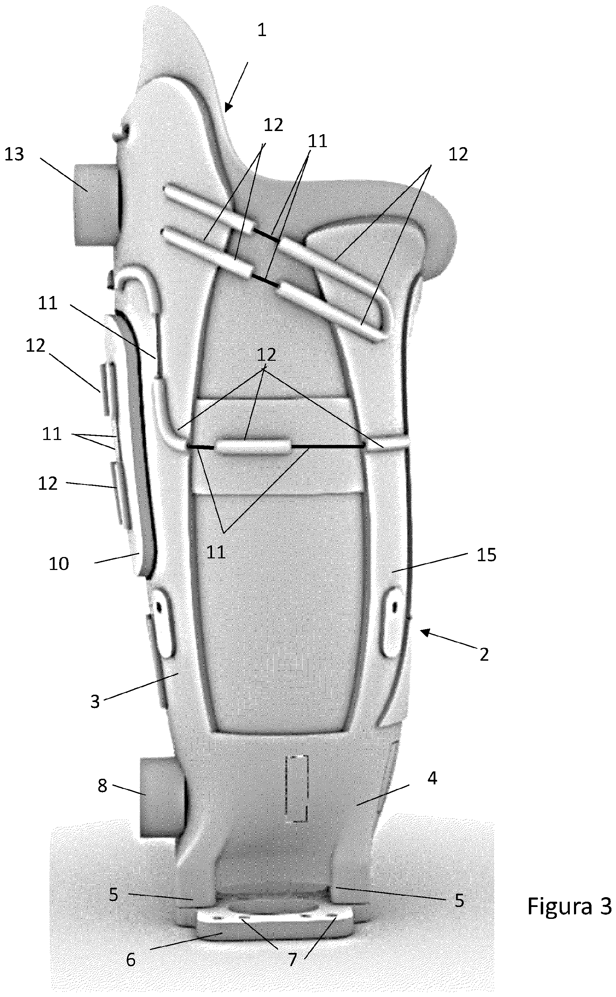

Figure 3 shows a rear view of the terminal coupling to a stump according to the previous figures.

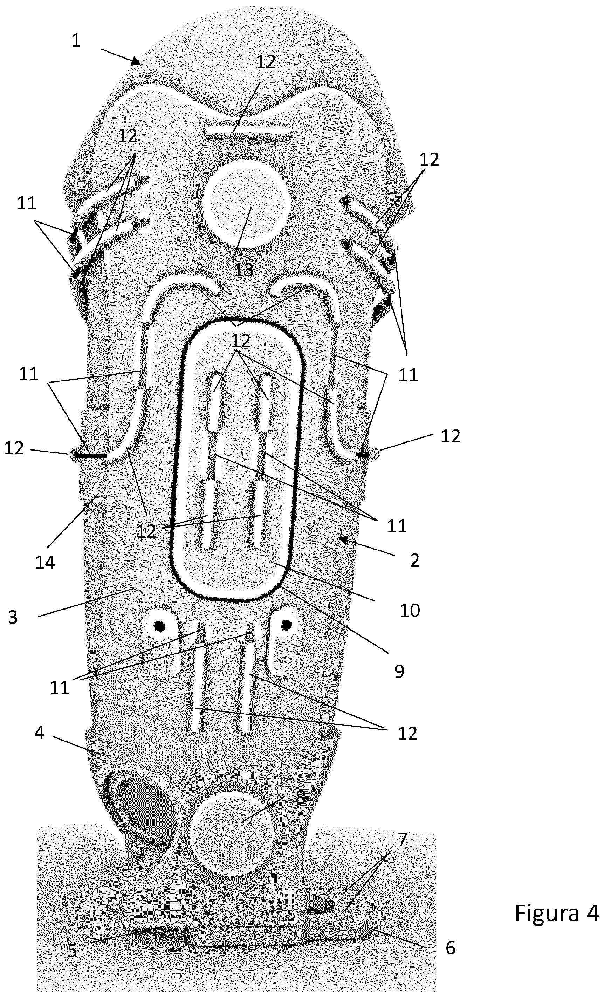

Figure 4 shows a side view of the terminal coupling to a stump in the previous figures.

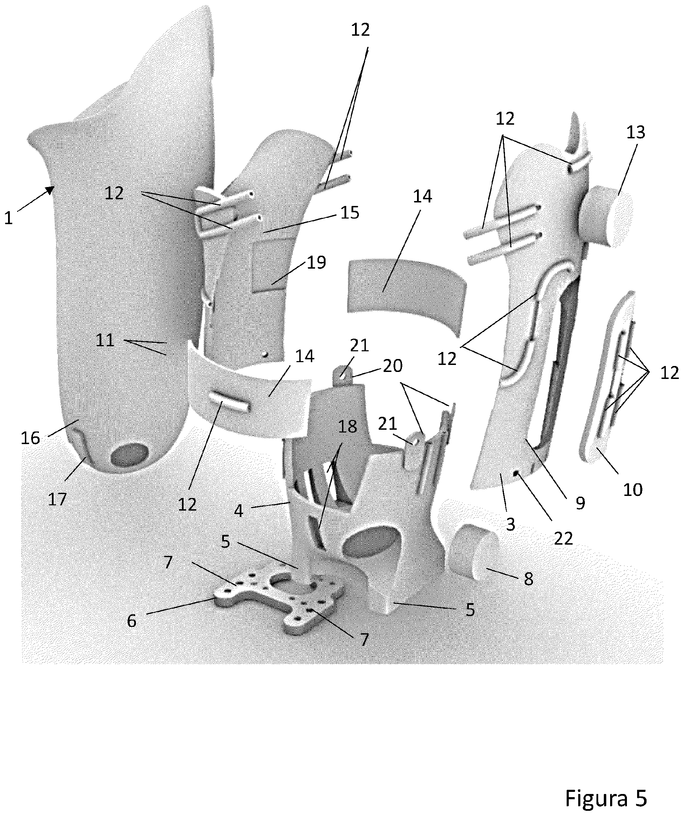

Figure 5 shows a decomposed view of the terminal coupling to a stump according to the previous figures in which the support structure is exploded into the different elements of which it is made.
Figure 6 shows a bottom view of the one stump coupling terminal showing first and second component and the connection plate.

The fig. 7 shows an example of an interface for entering the data detected on a stump and necessary for the conformation of the internal surface of the socket in a way corresponding to the morphology, i.e. the shape, of the external surface of the stump by means of a parameterized algorithm which calculates a function describing the shape of the said external surface of the stump and therefore of the internal one of the socket.

### Detailed description of the figures

With reference to the figures, they show a preferred and non-limiting example of embodiment of the coupling terminal to a stump of a transfemoral prosthesis according to the present invention. In this example the features expressed in a general way in the introduction are implemented with specific elements and devices which are to be considered as non-limiting examples of the more general definitions of the present description.

The figures show a coupling terminal to a stump of a transfemoral prosthesis. The said prosthesis includes said coupling terminal which can be fixed by means of an adjustable connecting element, separable to the upper end of an artificial limb which replaces the missing part of the natural limb. Said coupling terminal is of the type anatomically adapted or adaptable to the stump and includes a first component 1 shaped as a socket which is configured to house a stump inside it and which is open at an upper end and is internally closed with a concave surface corresponding to a convex surface externally at its lower end.

In particular, the coupling terminal can be fixed using an intermediate plate 6 which guarantees the orthopedic technician the possibility of aligning the coupling terminal along the x and y axis, and the replacement limb of the missing limb in an optimal manner both in conditions in which the patient is static and in conditions in which the patient is dynamic. These operations of alignment optimization can take place both in the initial setting phase and in the maintenance phase over time.

The said first component 1 is shaped externally so as to be housed in the internal containment compartment of an external support structure 2 which constitutes a second structural component of the said terminal, and which external structure presents at least one upper opening for insertion of the said first component 1. At its lower end, the said second component 2 is also provided with a concave internal surface and a substantially convex or domed external surface as indicated with 4.

The said first component 1 is made of a flexible material which can be processed, for example but not limited to, by additive layer deposition devices such as a 3D printer or the like. The support structure 2, however, is made of a relatively rigid material, i.e. less flexible than the first component 1, in particular a plastic material.

While the first, more flexible component 1 is substantially made up of a single piece, the second component, i.e. the more rigid support structure 2, is made up of a plurality of elements 3, 10, 14, 15, 6 which are connected or can be connected to each other so as to form together an external covering of the first component 1, which overlaps at least some areas of the external surface of the said first component, preferably which extends over a predominant part of the external surface of the said first component 1, and in which the said elements 3, 10, 14, 15, 6 are connected to each other so as to be able to be brought closer and further apart from each other and/or to be able to vary a mutual angular orientation according to at least one axis, thus varying the volume of accommodation in the compartment provided by said second component 2 for the said first component 1 and generating compressions in some areas and/or according to some directions of the socket or reducing the said compressions even to a value equal to zero.

As it will appear more clearly from the following description, the said elements which constitute the rigid support structure 2 can be connected to each other, all or even in groups comprising two or more different elements, by means of tie rods which are provided in combination with manual traction members of the said tie rods in the direction of mutual approach of the elements which are connected to each other by them. The said traction members can also be operated in the opposite direction, i.e. in the sense of a reduction or cancellation of the traction on the tie rods to allow a mutual distancing of the elements connected to each other by the said tie rods 11, or a loosening of the compression of the support structure 2 or at least of some of the elements that compose it against the external surface of the first component 1.

As will appear more clearly from an exemplary embodiment illustrated in the figures, the elements are configured to exert compression forces on the first component 1 of flexible material and consequently against the stump housed in it, which forces act in pre-established areas of said first component 1 and/or which forces exert different types of compression actions, such as for example circumferential compressions and in other directions on said first component 1 and therefore on the stump.

In the exemplary embodiment illustrated in the figures, the support structure 2 includes a closed bottom element 4 which in the said specific embodiment is shaped like an inverted dome or cup and which has a concave internal surface. The lower edge of a lateral leg element indicated with 3 and an opposing medial leg element 15 are hinged to said bottom element 4. The two leg elements 3 and 15 have an angular extension in the circumferential direction smaller than the entire circumferential extension of the external surface of the first component 1 and therefore, in the condition mounted on the bottom element 4 and in use, the front and rear edges of the said two leg elements 3 and 15 are spaced apart from each other, leaving a substantially vertical axial band of the socket free on the two sides of said first component 1 which axial bands are substantially parallel to the sagittal plane of the user of the coupling terminal to a stump of the prosthesis and/or predominantly oriented parallel to said sagittal plane.

The said two leg elements 3 and 15 are connected to each other in correspondence with a substantially circumferential band provided in the area of the upper half of the support structure 2, i.e. the half associated with the open side of the same and opposite to the bottom element 4, by means of tie rods 11 for variable tightening of the said two elements against the first component 1 and therefore against a stump housed in the same. Preferably, said circumferential band is provided at the upper end of the support structure 2.

According to the embodiment of the illustrated example, said tie rods 11 are made up of flexible elements such as cords, strings, cables, ribbons or similar which are guided on a path coinciding with said substantially circumferential band and are coupled with their ends to members 13 which allow to exercise manually a traction force on them.

In the embodiment of the specific example, a single tie rod 11 is provided which is connected with one end to a winding core of a winding/unwinding device 13 and which is passed from said device 13 mounted on the lateral element 3, along guides 12 up to a return guide on the medial element 15, from there again towards the lateral element 13 and around the lateral element 3 in the direction of the opposite side and through a guide 12 up to a return guide on the said opposite side of the medial element 15 until it is fixed with its other end to the core of the said winding/unwinding device 13, in such a way that a rotational operation in one direction of the said core wraps the tie rod around it, generating a tensile tension in the tie rod and a rotation of the core in the opposite direction unwinds the tie rod from it, lengthening it and therefore loosening the traction action of the two lateral and medial elements 3, 15 against each other.

Thanks to the above features, a possibility of dimensional adjustment of the socket is generated in relation to a substantially medio-lateral, or sagittal, direction of the same. Therefore, it is possible to widen or narrow the socket in these directions depending on the dimensional variations that the stump performs, over time, following the different stress conditions of the same.

It appears evident that the said variation in position of the two front and rear elements does not consist in a translation of the said two elements reciprocally one with respect to the other and parallel to themselves, but since the lower end of the lateral and medial elements respectively is hinged to the bottom element 4, the said two parts essentially perform an oscillatory movement, i.e. along an angular path having the corresponding lower edge as the fulcrum.

According to a further feature, the support structure 2 includes further elements which are made up of independent parts separated from the lateral and medial elements and also from the bottom element and which in the illustrated executive example include a sector or an area of the lateral element 3 in which an opening 9 is provided and a wall element 10 which completes the wall of the lateral element 3 and which has a shape and extension substantially corresponding to those of the opening 9. The sector 10 of the lateral element 3 is made in the form of a piece separated from the lateral element 3 and can be moved parallel to itself in the direction of the axis of the opening 9, i.e. substantially perpendicular to the lateral element 3, and is connected to the lateral element 3 thanks to a tie rod 11 which is guided along a pre-established path so as to bring both ends of the same in correspondence with the winding and unwinding core of a second winding/unwinding device indicated with 8.

The path of the said tie rod 11 requires that it extends, starting from the core of the winding/unwinding device 8, upwards, in a vertical direction along the said wall segment 10 of the lateral element 3 and that once it has passed the upper edge of the said wall segment 10 it is deflected in a circumferential direction towards the medial element 15 and crosses it completely along a circumferential band which is substantially in a central position with respect to the open upper end of the socket 1 and the support structure 2 and at the closed lower end of the socket and/or the support structure 2.

In correspondence with the aforementioned circumferential passage band of the tie rod 11 and on both sides of the coupling terminal to a stump of the prosthesis, a separate element 14 is provided for connecting the facing rear front edges of the lateral-medial elements 3, 15. The said element 14 is interposed between the said rear front edges forming a sort of bridge which crosses the said vertical slot which distances the lateral element 3 from the medial element 15 on both said sides of the coupling terminal to a stump of the prosthesis which are oriented parallel or which have a predominantly parallel orientation to the sagittal plane, preventing the tie rod from coming into contact with the first component 1.

According to the illustrated embodiment, the said element is in the shape of an arched plate which carries on the front and rear side a guide segment 12 which constitutes a connection segment between the guide elements 12 on the lateral element 3 and those which medially surround the medial element 15.

According to yet another feature, the said element 14 in the form of an arched plate has ends respectively facing the lateral element 3 and the medial element 15, which slide into guides 19 made in the form of depressions or grooves in the wall of the corresponding side of the lateral element 3 and the medial element 15. Preferably the said guides are provided on the internal side of the lateral element 3 and the medial element 15 facing the external surface of the first component 1.

Therefore, these elements not only support the tie rod 11 preventing it from coming into contact with the wall of the first component 1, but at the same time constitute a sliding guide in the direction of mutual approach or separation of the two elements respectively lateral 3 and medial 15 on the two sides of the coupling terminal to a stump of the prosthesis in which the axial bands of the said first component are provided which are not covered by the said lateral and medial elements 3, 15.

In order to allow relative sliding between said arcuate plates 14 and the corresponding guides in the lateral 3 and medial 15 elements, the plates have a substantially rectangular plan shape with at least one upper and one lower edge substantially parallel to each other and spaced to the extent corresponding to the corresponding upper and lower edges of the guides 19.

In the specific embodiment illustrated, the tie rod is fixed with one end to the core of the winding/unwinding device 8 and the opposite end is brought back to said core by passing the tie rod along a pre-established path defined by the guides 12. In particular, the tie rod departs from the core and continues upwards passing through the guides 12 integral with the wall segment 10 of the lateral element 3. Subsequently the guides are shaped so as to deflect the tie rod 11 from the straight path upwards in an S-shaped path that ends with a curve whose exit branch is oriented in a substantially circumferential direction and towards the medial element 15.

At the circumferential position of the exit branch of the said S-curve, the plate 14 is provided with a guide segment 12 for the tie rod which is also oriented circumferentially, and which guides the tie rod 11 towards the guides provided along the medial side of the medial element 15. Said guides carry the tie rod on the opposite side of the coupling terminal to a stump of the prosthesis in correspondence with which side a second plate 14 is provided, which second plate 14 is diametrically opposite to the first plate 14 and which second plate 14 also carries a guide segment 12 oriented circumferentially, while the said guide segment on the plate 14 is continued with an S-shaped segment having an entry branch coinciding with the guide 12 on the plate 14 and having an exit branch oriented in a substantially vertical direction and aligned with further vertical guides 12 provided on the wall segment 10. Through these guides, the tie rod is carried to the winding/unwinding device located underneath the wall segment 10 and to whose core its end is fixed, in order to allow the exertion of displacement forces of the relative parts described above thanks to a traction of the tie rod through a winding action exerted manually with the knob 8.

By wrapping the tie rod 11, the side wall segment 10 is urged to penetrate to a greater or lesser extent inside the support or load-bearing structure and against the first component 1, while circumferentially a further compressive force of the said first component 1 is exerted against the stump. The greater or lesser penetration of the wall segment 10 into the shape of the opening 9 generates a greater or lesser compression of the said wall sector 10 against the corresponding area of the first component 1 and against the corresponding area of the stump.

There is therefore a very localized action of compression on the flexible component and therefore on the stump, in combination with an action of greater or lesser circumferential constriction of the said first component against the stump.

The area on which the wall segment 10 exerts its action is provided in the central area of the lateral element 3 with reference to the vertical and horizontal extension for which this action is localized on the area of the stump or of the first component 1 coinciding with it.

According to yet another feature which is advantageously represented here in combination with one or more of the embodiment and variants described, the bottom element 4 can be fixed to the remaining part of the prosthesis, or to an artificial limb replacing the part of the missing natural limb by means of an intermediate plate 6. Said plate can in turn be fixed to the supporting structure 2, or to the lower side of the bottom element 4 and this fixing is carried out in such a way as to allow the adjustment of the position of the support structure 2 with respect to the further part of the artificial limb resting the prosthesis on the ground.

In general, said plate 6 can be fixed to said support or load-bearing structure 2 and/or to the further part where it rests on the ground by means of sliding slides oriented in two directions orthogonal to each other and contained in the horizontal plane.

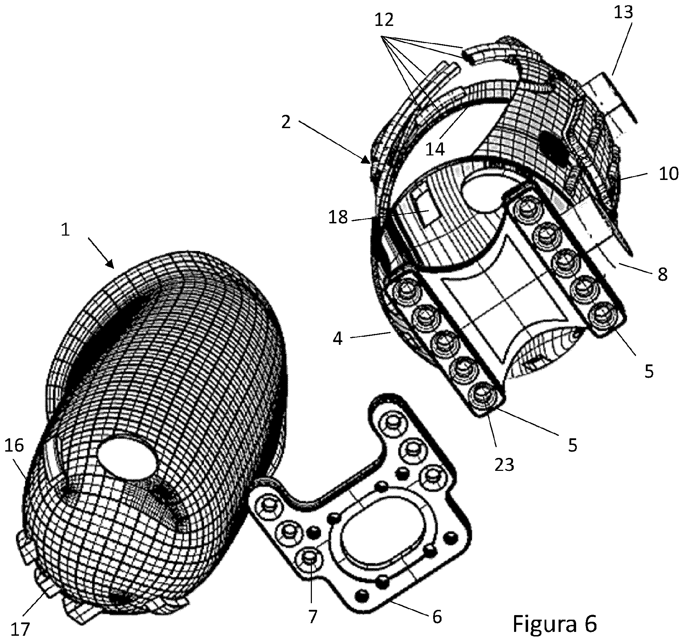

Alternatively and as shown in the example illustrated and specifically in figure 6, the plate 6 is provided with two rows of holes which are oriented in two directions, while on the lower side of the bottom element 4 of the support or load-bearing structure 2 a similar row of holes is provided, and the said plate is fixed by bolts or tightening screws alternately with one of the holes to at least one of the holes provided on the lower top of the supporting structure 2, allowing the plate 6 to assume different positions parallel to said row of holes depending on the choice of holes on the plate 6 and on the supporting structure 2, or on the base terminals 5 which have the said row of holes.

As is evident, the plate 6 has two rows of holes parallel to each other and spaced laterally and which rows of holes are foreseen to coincide with the respective rows of holes on fixing terminals 5 provided at the lower end of the supporting structure 2.

Preferably the two rows of holes, i.e. the terminals 5, are substantially coincident with the lateral walls oriented in a sagittal or predominantly sagittal direction, the corresponding rows of holes on the plate 6 being spaced to correspond to each other.

The adjustment in a direction perpendicular to that of the rows of holes can take place thanks to the provision of similar configurations of cooperating rows of holes on the plate 6 and on the fixing terminals 5 which are oriented according to a different direction or the said further rows of holes are provided to couple with the holes of corresponding rows of holes provided in a terminal of the artificial limb part of the prosthesis. It should be noted that this part of the artificial limb is not illustrated in detail and that it can consist of a simple rigid rod or a device such as a robotic limb or an artificial limb having some functions similar to the natural limb, which artificial limb is fixed to the stump by means of the coupling terminal of the aforementioned prosthesis. Thanks to the coupling with the possibility of adjustment, according to at least two directions in the horizontal plane, of the said artificial limb with the said coupling terminal to a stump, it is possible to align the artificial limb to the stump so that the various mobility axes provided by the artificial limb are correctly centered, or are correctly positioned in relation to the stump to guarantee the most natural stress possible on the user's musculoskeletal system and in particular on the femur and hip.

With reference to the first component 1 of flexible material, the said component comprises an internal surface forming the containment for the stump and an external surface separated by a certain thickness of the said component. The conformation of the internal surface thereof and/or also the conformation of the external surface thereof is/are obtained with a specific method of generating a 3D virtual model of the said surface, which model is constituted by a representation function of the said surface/surfaces calculated by means of a parameterized algorithm.

According to an improvement of the said method, the generation of the 3D virtual model, i.e. the said function calculated by means of the said algorithm, also provides the conformation of the internal surface and optionally of the external surface of the second component 2 in such a way that said internal surface of the second component 2 fits at least in some areas with the external surface of the said first component at least at the areas at which the said second component 2 covers the said first component 1. In addition, the said function calculated by means of the said algorithm, also provides the arrangement of the members, i.e. of the winding/unwinding devices 8 of the tie rods, as well as the paths of the said tie rods along the said support structure 2 and/or optionally also at least variants of the elements that form the said support structure.

According to a further feature, the said first component 1 of flexible material and the said second more rigid component constituted by the support structure 2 may show mutual engagement elements that allow a centering of the said first component 1 with respect to the said second component 2. Preferably, said elements are provided in the area of the bottom element 4 of the supporting structure 2 and of the corresponding closed lower end 16 of the first component 1.

In the illustrated embodiment, along the shell wall of the lower end, the first component 1 has one or more projections 17 which protrude outward from said external shell surface and which are intended to engage in corresponding niches or through cutouts 18 of the bottom element 4 of the supporting structure 2.

In a preferred embodiment, the projections 17 on the first component 1 and the niches or cutouts 18 provided in the bottom element 4 of the supporting structure 2 have corresponding plan shapes and substantially corresponding dimensions, or rather the projections have dimensions slightly smaller than those of the niches or cutouts 18, so that in the condition of mutual engagement of the said projections 17 in the said niches or in the said cutouts 18, there is a precise relative positioning of the first component 1 with respect to the second component, i.e. to the support structure 2 and that said positioning is also stable.

Again according to an embodiment, the fixing of the lateral 3 and medial 15 leg elements to the bottom element 4 to guarantee a correct relative positioning of the said parts, provides that the bottom element has a plurality of extensions 20 which protrude beyond the separation edge of the bottom element with respect to the lateral and medial leg element 3, 15 and which are distributed in a certain number along the extension of the said separation edge, while the said extensions 20 are provided with through holes 21 and while the lateral 3 and medial 15 leg elements have holes 22 which are provided in positions coinciding with the holes 21 of the said extensions 20 in the mounted condition of the said leg elements 3 and 15 to the bottom element 4. The said leg elements 3 and 15 are fixed to the bottom element 4 by means of transverse pins which engage in the said coinciding holes 21 and 22.

According to a feature relating to the design and production steps of the said coupling terminal for the stump to the artificial limb replacing the missing limb, the present invention provides a method which allows to generate, by means of a calculation algorithm, a virtual model of the surface of the stump and therefore of the internal surface and/or of the corresponding external surface of the first component of flexible material 1, i.e. a function which describes the three-dimensional shape of the said surface/surfaces on the basis of pre-established dimensional parameters of the stump which are easy to measure on the stump itself and which function provides at the same time the configuration of an external shell superimposed on the first component 1, which shell constitutes a second component of more rigid material which is in the form of a supporting structure made up of a plurality of variable compression elements. These can be moved relatively to each other according to one or more different degrees of freedom and are provided in combination with one or more relative movement members such as, for example, the winding/unwinding devices 8 of one or more tie rods 11 described in the example.

It is possible to envisage different types of model calculation algorithms. In an embodiment, the algorithm consists of a machine learning algorithm that has been trained on the basis of existing data relating to the shape of the external surface of the stump and the parameters identified to specifically define the shape of the said surface for a specific stump and therefore for the related socket anatomically adapted to the said stump.

The machine learning algorithm thus trained presents as input variables the one or more parameters defined to characterize a specific stump and on the basis of this calculates the three-dimensional model or function representing the three-dimensional conformation of the said external surface of the stump and therefore of the internal surface of the socket, i.e. the internal and/or the external surface of the first component 1 and/o the internal surface of the second component 2.

An embodiment of the method provides as parametric input variables of the algorithm for the specific configuration of the external three-dimensional surface of the stump and consequently of the internal three-dimensional surface of the socket, i.e. the internal and/or the external surface of the first component 1 and/o the internal surface of the second component 2, the dimensional parameters relating to the circumferential measurements at the different heights of the stump, i.e. at different levels in the vertical direction, the longitudinal extension in the vertical direction and some circumferential measurements along circumferences that lie on planes inclined with respect to the horizontal plane or perpendicular to the longitudinal extension of the stump, thickness of the stump measured with a caliper at pre-established points of its axial extension, any inclinations of the circumferences with respect to the horizontal orientation and furthermore the user's height and weight as well as optionally also one or more parameters relating to the anchoring device 6 of the socket to the artificial limb.

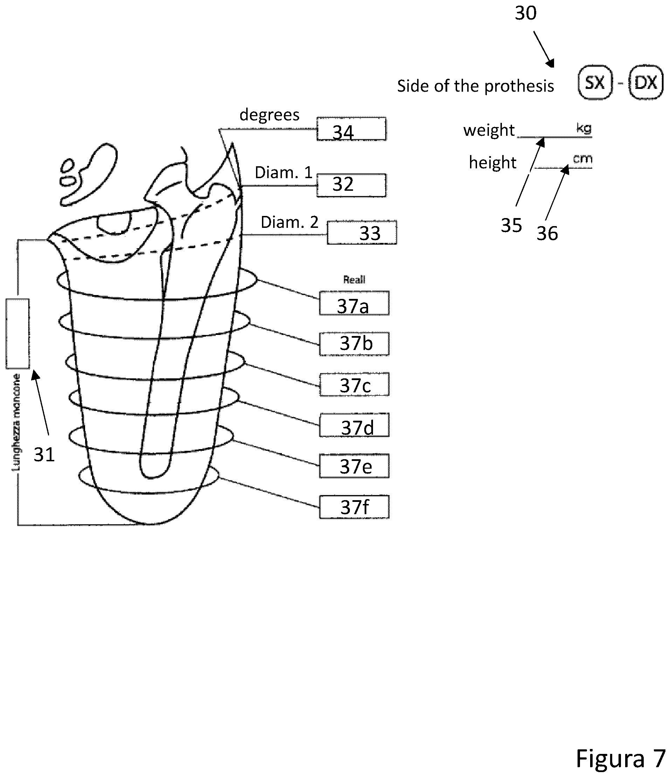

Fig. 7 shows an example of an interface for entering the data detected on a stump and necessary for the conformation of the internal surface of the socket i.e. the internal and/or the external surface of the first component 1 and/o the internal surface of the second component 2, in a way corresponding to the morphology, i.e. the shape, of the external surface of the stump by means of a parametrized algorithm which calculates a function describing the shape of the said external surface of the stump and therefore of the internal one of the socket, i.e. the internal and/or the external surface of the first component 1 and/o the internal surface of the second component 2. This interface can be in the form of a paper form or also in the form of a graphical interface displayed on a screen and in which the indicated fields are data entry windows using input devices such as keyboards, touch functionality of a display, voice commands and other data entry methods.

In the data collection interface relating to the stump of a specific user, said interface requires the entry of the indication whether it is the right or left leg as indicated with 30. Field 31 requires the entry of the measurement of the length of the stump in the vertical direction. Fields 35 and 36 relate to the indication of the height and weight of the subject for whom a socket must be created, configured in an anatomical way corresponding to the shape of the external surface of the stump.

Fields 32 and 33 relate to the measurements of the diameter along planes inclined with respect to the horizontal plane or to a plane perpendicular to the longitudinal axis of the stump and the indication of the angle of inclination 34 with respect to said horizontal direction or perpendicular to the longitudinal axis of the stump.

Fields 37a to 37f require indications of the circumferences or diameters at different heights, or at different levels along the longitudinal axis of the stump and according to horizontal or perpendicular planes to said longitudinal axis.

The said data are provided to the algorithm which, as indicated above, generates a three-dimensional, virtual model of the external surface of the stump and therefore of the corresponding internal surface of the socket according to the above definition, i.e. a function that represents the said three-dimensional surfaces in space.

This three-dimensional model or said functions are converted into a file compatible with a three-dimensional printer or other manufacturing device to which said converted file is supplied for the production of the anatomically adapted model of said socket by means of said 3D printing technique.

The final production step consists of assembling the support structure 2 and coupling it to the first component 1 of flexible material to obtain the finished socket, as well as fixing the lower end of said supporting structure 2 to the remaining part of the prosthesis comprising one of the different types of artificial limbs replacing the missing part of the natural limb.

Finally, it should be highlighted that although the invention has been described with reference to a type of prosthesis of the type called trans-femoral, the characteristics of the invention apply to any type of prosthesis intended to replace a part of the missing limb relating to the upper and/or lower limbs and in which a terminal is provided for coupling to the remaining part of the limb, or to the stump to which a specific artificial limb can be fixed thanks to an adjustable joint, the configuration of the present invention not having to be modified except for obvious adaptations to the corresponding stump morphology.

## Claims

1. Method of designing a trans-femoral prosthesis, which trans-femoral prosthesis includes an artificial limb replacing the missing limb and a terminal for coupling said artificial limb to a stump, which terminal is in the form of an anatomically shaped socket on the stump and in which the said stump is housed and which can be fixed to the upper end of the said artificial limb, which method involves the following steps:
the separation of the coupling terminal to the stump into two functional components (1, 2) which can be coupled and/or separated from each other and of which,
a first component (1) of flexible material and a second component which is constituted by a support structure (2) of rigid or less flexible material than that of the first component and which second component has a housing cavity for said first component of flexible material;
providing said first component (1) with a shape defining a cavity delimited by a wall which has an internal surface and an external surface spaced one from the other by a thickness of the said wall;
the housing cavity of the said second component (2) for the said first component (1) having an internal surface corresponding to the shape of at least some areas of the external surface of the said first component (1), at least some of the said areas of the external surface of the said first component (1) being provided for being overlapped by the said internal surface of the said second component (2);
defining an algorithm that describes the three-dimensional trend of the shape of the internal surface of the first component (1) intended to adhere to the external surface of a generic stump on the basis of descriptive parameters of pre-established dimensional sizes of said stump;
generating a function that describes the shape of the internal surface of said first component (1) morphologically adapted to a specific stump, applying said algorithm to values of said descriptive parameters of said pre-established dimensional quantities measured on said specific stump;
generating a digital model of the internal surface and of the external surface of at least the said first component (1) of flexible material and/or of the internal surface of said second component (2) of more rigid material at the said at least some areas basing on the said function, which digital model is configurable or convertible into a command file for the control of a numerically controlled manufacturing device, such as a 3D printer or chip removal machine, or the like,
the manufacturing of said prosthetic socket obtained by executing said command file;
the coupling of the said two components (1, 2) of the said prosthetic socket, i.e. the support structure (2) of rigid or less flexible material and the first component (1) of flexible material;
the fixing to said supporting structure (2) to an artificial substitute for the missing limb.

2. Method according to claim 1, in which the said rigid or less flexible supporting structure (2) is realized in the form of a deformable element relative to a dimensional variation in at least one, preferably in a plurality of pre-established directions relative to a longitudinal axis of the said rigid support or load bearing structure.

3. Method according to claims 1 or 2, which method involves the use of an algorithm that describes the shape of the external surface of the stump on the basis of dimensional parameters of said stump.

4. Method according to one or more of the previous claims, wherein the algorithm is configured to calculate the shape of the external surface of a specific stump and therefore of the corresponding internal surface of the prosthetic socket, namely the internal surface of the first component (1) and/or the external surface of the said first component (1) and/or the internal surface of the housing cavity of the said second component (2) for the said first component (1), on the basis of the dimensional values of a stump relative to one or more of the following parameters: right or left limb, length of the stump, measurement of the thigh, circumference of the stump, thickness of the stump measured with a caliper at pre-established points of its axial extension, any inclinations of the circumferences with respect to the horizontal orientation and further of the height and weight of the user and/or further one or more parameters relating to the anchoring device (6) of the socket to the artificial limb.

5. Method according to one or more of the previous claims, wherein the support or load bearing structure includes elements for variable radial compression of the flexible socket against the stump, which are provided in at least one circumferential band of the said stump and/or compression elements localized of at least a partial area of the prosthetic socket against the stump and whose elements are connected to each other by means of relative displacement members that can be controlled manually in the direction of mutual approach and in the direction of mutual distancing, the said algorithm being configured to calculate the position of the members of mutual displacement of the localized compression elements that form the said support or load bearing structure (2) and an initial compression condition, while a manual control of the compression condition is provided by activating the said mutual displacement members (8) in order to manage the volumetric change during the day, with the initial compression condition calculated by the algorithm defined to such an extent as to regulate the vascular and lymphatic pressure of the stump, to prevent it from swelling and with the compression condition being adjusted manually in order to improve comfort of the fit.

6. Method according to claim 5, wherein said relative displacement members are constituted by tie rods and/or strings which are sent back to at least one mechanism for winding and/or unwinding said tie rods on a reel, optionally a single tie rod which is guided along the structure and connects all the elements that make up the said structure together in succession.

7. Method according to one or more of the previous claims, in which an organ is provided for regulating the relative coupling position of the said socket with the said artificial limb, which organ allows the relative position of the said socket and the said artificial limb to be varied according to at least two degrees of freedom, according to at least two directions in the horizontal plane.

8. Prosthesis manufactured with a method according to one or more of the preceding claim 1 to 7, wherein the prosthetic socket is formed by the combination of:
a first component (1) of flexible material and a second component which is constituted by a support or load bearing structure (2) of rigid or less flexible material than that of the first component and which second component has a housing cavity for said first component of flexible material;
said first component (1) being provided with a cavity delimited by a wall which has an internal surface and an external surface spaced one from the other by a thickness of the said wall;
said housing cavity of the said second component (2) for the said first component (1) having an internal surface corresponding to the shape of at least some areas of the external surface of the said first component (1), at least some of the said areas of the external surface of the said first component (1) being provided for being overlapped by the said internal surface of the said second component (2);
said second component (2) is provided with at least one upper opening of the said housing cavity for insertion of the said first component (1) and with a closed bottom element (4) having a concave internal surface;
said second component (2) further comprising a lateral leg element (3) and an opposing medial leg element (15) which are hinged at their lower edge to the upper edge of said bottom element (4);
said two leg elements (3, 15) having an angular extension in the circumferential direction smaller than the entire circumferential extension of the external surface of the first component (1);
said two leg elements (3, 15) being connected to each other at a substantially circumferential band provided in the area of the upper half of the support or load bearing structure (2) by means of tie rods (11) combined with traction organs (13) for variable tightening of the said two leg elements one against the other and against the first component (1) and by means of circumferential guiding plates (14) engaging both leg elements in a circumferentially sliding way by means of sliding guides (19) provided on the said leg elements (3, 15) at the mutual facing edges of the said leg elements, said guides being preferably provided on the internal side of the said leg elements (3, 15) facing the external surface of the first component (1) and
the tie rods (11) are passing on the said circumferential plates (14) with circumferential sections thereof.

9. Prosthesis according to claim 8, **characterized in that** the second component (2), i.e. at least the said leg component (3, 15), are composed of several elements separated from each other and joined by one or more mutual clamping elements, of which one or more mutual clamping elements are connected to one or more manually controlled shortening and/or lengthening members and in which optionally
said one or more members can be constituted for example by one or more winding cores of at least one of the said clamping elements and the said clamping elements are in the form of one or more flexible tie rods.

10. Prosthesis according to one or more of the previous claims 8 or 9, in which said first component (1) of flexible material and the said second more rigid component constituted by the support or load bearing structure (2) are provided with mutual engagement elements for centering said first component (1) with respect to the said second component (2) which are preferably provided in the area of the bottom element (4) of the support or load bearing structure (2) and of the corresponding closed lower end (16) of the first component (1).

11. Prosthesis according to one or more of the previous claims 8 to 10, in which an insert is provided between the support or load bearing structure (2) and the artificial limb for regulating the relative alignment position of the said support or load bearing structure and the said artificial limb, which adjustment insert allows a relative movement of the said two parts at least in a substantially horizontal plane.
